# EUROPEAN PATENT SPECIFICATION

(11) **EP 2 149 377 B1**
(45) Date of publication and mention of the grant of the patent: **14.09.2016**
(21) Application number: 08171362.0
(22) Date of filing: 11.12.2008
(51) Int. Cl.: A61K 36/29, A61P 3/10, A61K 36/28, A61K 36/16, A61K 36/38, A61K 36/258, A61K 36/82, A61K 36/88, A61K 36/752, A61K 36/00, A61K 36/68, A61K 36/20, A61K 36/24, A61K 36/48, A61K 36/9066, A61K 9/00, A61K 9/68, A61K 9/20, A61K 9/28, A61K 9/48, A61K 9/24

(54) **Compositions containing berberine or extracts containing it, for the prevention and treatment of alterations of the lipid and carbohydrate balance**
Zusammensetzungen mit Berberin oder Extrakten damit zur Vorbeugung und Behandlung von Störungen des Fett- und Kohlenhydrat-Gleichgewichts
Compositions contenant de la berbérine ou des extraits la contenant, pour la prévention et le traitement des altérations de l'équilibre des lipides et des hydrates de carbone

(30) Priority: 29.07.2008 IT MI20081403
(43) Date of publication of application: 03.02.2010
(73) Proprietor: Velleja Research SRL, 29010 Pontenure (PC) (IT)
(72) Inventor: Di Pierro, Francesco, 29010, PONTENURE (PC) (IT)
(74) Representative: Minoja, Fabrizio

(56) References cited:
- WO-A-2007/035515
- DATABASE WPI Thomson Scientific, London, GB; AN 2008-G34632 XP002524418 ZHANG B: "Chinese medicinal preparation for hepatobiliary disease comprises drug effect component and/or auxiliary request" & CN 101 049 349 A (BEIJING YINKE RUISI BIOLOGICAL PROD RES INST) 10 October 2007 (2007-10-10)
- DATABASE WPI Week 200325 Thomson Scientific, London, GB; AN 2003-248897 XP002524420 & CN 1 362 216 A (YANG M) 7 August 2002 (2002-08-07)
- STERMITZ F R ET AL: "5'-Methoxyhydnocarpin-D and pheophorbide A: Berberis species components that potentiate berberine growth inhibition of resistant Staphylococcus aureus" MEDICINAL & AROMATIC PLANTS ABSTRACTS, RESOURCES, NEW DELHI, INDIA - NEW DELHI, vol. 23, no. 5, 1 October 2001 (2001-10-01), XP018016607 ISSN: 0250-4367
- YIN J ET AL: "Efficacy of berberine in patients with type 2 diabetes mellitus" 1 May 2008 (2008-05-01), METABOLISM, CLINICAL AND EXPERIMENTAL, W.B. SAUNDERS CO., PHILADELPHIA, PA, US, PAGE(S) 712 - 717 , XP022624576 ISSN: 0026-0495 [retrieved on 2008-04-26] * the whole document *
- LUJIANG YUAN ET AL: "Hypoglycemic and Hypocholesterolemic Effects of Coptis chinensis Franch Inflorescence" PLANT FOODS FOR HUMAN NUTRITION, KLUWER ACADEMIC PUBLISHERS, DO, vol. 61, no. 3, 21 September 2006 (2006-09-21), pages 139-144, XP019437364 ISSN: 1573-9104
- CHHETRI D R ET AL: "Antidiabetic plants used by Sikkim and Darjeeling Himalayan tribes, India" JOURNAL OF ETHNOPHARMACOLOGY, ELSEVIER SCIENTIFIC PUBLISHERS LTD, IE, vol. 99, no. 2, 3 June 2005 (2005-06-03), pages 199-202, XP025270100 ISSN: 0378-8741
- AKHTAR M S ET AL: "Hypoglycaemic effect of Berberis aristata roots, aqueous and methanolic extracts in normal and alloxan-diabetic rabbits" PHARMACOLOGYONLINE,, vol. 2, 1 January 2008 (2008-01-01), pages 845-856, XP009115649
- GAZAK RADEK ET AL: "Silybin and silymarin - New and emerging applications in medicine" CURRENT MEDICINAL CHEMISTRY, BENTHAM SCIENCE PUBLISHERS BV, BE, vol. 14, no. 3, 1 January 2007 (2007-01-01), pages 315-338, XP009115597 ISSN: 0929-8673
- MAGHRANI M ET AL: "Study of the hypoglycaemic activity of Fraxinus excelsior and Silybum marianum in an animal model of type 1 diabetes mellitus" JOURNAL OF ETHNOPHARMACOLOGY, ELSEVIER SCIENTIFIC PUBLISHERS LTD, IE, vol. 91, no. 2-3, 1 April 2004 (2004-04-01), pages 309-316, XP009115595 ISSN: 0378-8741
- HUSEINI H FALLAH ET AL: "The efficacy of Silybum marianum (L.) Gaertn. (silymarin) in the treatment of type II diabetes: a randomized, double-blind, placebo-controlled, clinical trial" PHYTOTHERAPY RESEARCH, JOHN WILEY & SONS LTD. CHICHESTER, GB, vol. 20, no. 12, 1 December 2006 (2006-12-01), pages 1036-1039, XP009115663 ISSN: 0951-418X [retrieved on 2003-10-30]

## Description

The present disclosure relates to compositions containing berberine in association with extracts or compounds of plant origin which enhance its bioavailability.

### Prior art

Cholesterol is a molecule which plays an essential physiological role: it prevents excessive fluidity of the cell membranes, and is an important precursor in the synthesis of hormones, Vitamin D and bile salts.

In the blood, cholesterol is bound to proteolipid substrates with carrier functions. These carriers are distinguished according to their density. On the basis of this subdivision, cholesterol can be found in the form of kilomicrons, HDL (High-Density Lipoproteins), LDL (Low-Density Lipoproteins), VLDL and IDL (Very Low and Intermediate Density Lipoproteins respectively). Cholesterol, especially in the form of LDL, can undergo an oxidation process which endangers the arteries. Oxidised cholesterol tends to accumulate in the cells (in this case called Foam Cells) which constitute the inner wall of the arteries. The resulting thickening, which narrows the lumen of the artery, is called an atheromatous plaque. Said narrowing considerably limits the flow of oxygen-rich arterial blood, leading to atherosclerosis and its cardiovascular complications (heart attack, stroke, etc.).

Hypercholesterolaemia is often accompanied by a marked increase in blood triglycerides. Conditions characterised by plasma cholesterol levels exceeding 180-200mg/dl, and triglyceride levels exceeding 200 mg/dl, are therefore now more correctly described as dyslipidaemia.

A deterioration in the lipid balance can result from serious dietary disorders, liver metabolism disorders, and other diseases.

Conditions predisposing to an increase in LDL and triglycerides include diabetes mellitus, hypothyroidism, obesity, alcoholism, kidney disease, the use of oral contraceptives, liver disease and genetic disorders.

Two main classes of drugs are used to correct the altered lipid balance: statins and fibrates. Statins (e.g. atorvastatin, fluvastatin, pravastatin and simvastatin) act by inhibiting the enzyme 3-hydroxy-3-methylglutaryl coenzyme A reductase (HMG CoA reductase) involved in the biosynthesis of cholesterol. The use of statins has reduced mortality due to coronary disease: by normalising the blood cholesterol and consequently reducing the development of atherosclerotic plaques, cardiovascular events and mortality are also reduced. Fibrates are used in forms of dyslipidaemia in which triglycerides prevail. In addition to reducing the circulating levels of triglycerides, these substances increase the HDL fraction, modify the LDLs, making them less atherogenic, and reduce the fibrinogen levels. In order to thin the blood and prevent clot formation the treatment is often associated with platelet aggregation inhibitors such as low doses of acetylsalicylic acid, especially as secondary prevention after a thrombotic event, such as a myocardial infarction or stroke. Other drugs used to treat dyslipidaemia include probucol, nicotinic acid, niacin and derivatives thereof.

In addition to the established treatments, there are many compounds of natural origin which can supplement or replace the classic drugs listed above.

For example, red yeast rice (*Monascus purpureus*) inhibits the enzyme HMG-CoA-reductase, consequently acting as a natural statin.

Garlic, used in the form of standardised dried extract or essential oil, is useful in cases of hypercholesterolaemia due to its platelet aggregation inhibiting and fibrinolytic properties and its ability to improve the HDL/LDL ratio and reduce the triglyceride level.

Caigua is a plant native to South America which has been used to treat hypercholesterolaemia for centuries. Recent studies have demonstrated the ability of this plant to reduce LDL levels and increase HDL levels.

Chitosan is a polymer obtained from chitin, a substance extracted from the exoskeleton of shellfish. Its use reduces the absorption of dietary lipids by up to 30%.

Gugulipid is a resin obtained from an Indian tree, *Commiphora mukul,* used in ayurvedic medicine to treat hypercholesterolaemia by rebalancing the ratio between HDL and LDL and reducing the viscosity of the blood.

Policosanols, extracted from sugar cane or rice, effectively reduce cholesterol and triglycerides.

Artichoke (*Cynara scolymus*) leaf extract is a preparation which has been studied for its cholesterol-reducing properties. The active ingredient contained in the extract is believed to be luteolin-O-glucoside, a probable inhibitor of the reductase responsible for liver cholesterol synthesis.

It has been known for nearly 50 years that plant sterols and stanols, also called phytosterols and phytostanols, reduce the cholesterol level by partly inhibiting the absorption of cholesterol in the intestine. Sterols are found in nature in small amounts in many types of fruit, vegetables, nuts, seeds, pulses and vegetable oils. Finally, the polyunsaturated acids in the omega-6 series appear to reduce the blood cholesterol level by reducing the plasma LDL levels. However, this benefit seems to be partly attenuated by the fact that the same fatty acids simultaneously reduce HDL cholesterol. Omega-3 polyunsaturated fatty acids basically reduce the plasma levels of triglycerides, by interfering with their incorporation in the VLDLs in the liver. They consequently seem to possess an important antithrombotic action, as high levels of triglycerides in the blood reduce the fibrinolysis process.

Therapeutic approaches designed to improve the altered lipid balance have so far either proved partly ineffective or caused sometimes serious side effects.

Some authors recently analysed the efficacy of an alkaloid known as berberine obtainable from various medicinal plants used in Chinese medicine. Berberine is an isoquinoline alkaloid which, in the form of the sulphate and phosphate, has been used to treat malaria, bleeding and fever. According to more recent studies, this compound reduces the cholesterol level. Berberine is mainly extracted from *Coptis chinensis* and *Berberis aristata.* When taken orally for 3 months by 32 patients with high blood cholesterol levels it reduced plasma cholesterol by 29%, triglycerides by 35%, and LDL cholesterol by 25%. Researchers from the Chinese Academy of Medical Sciences in Beijing, using human hepatoma cells, have demonstrated that berberine increases the expression of LDLR (low-density lipoprotein receptor). Increased expression of LDLR by berberine takes place via a post-transcription mechanism which stabilises the messenger RNA. Berberine therefore potentially seems to be a new blood-lipid reducing agent with an action mechanism different from those of the statins and other conventional therapeutic compounds. In view of its high safety profile and tolerability, demonstrated clinically and by very long use in traditional Chinese medicine, berberine presents as an elective molecule in the treatment of hypercholesterolaemia. Moreover, in view of its blood-sugar reducing efficacy in diabetics, and the increasingly common concomitant alteration of blood sugar and lipid levels in patients suffering from obesity and/or type II diabetes, berberine is also an elective compound in the treatment of carbohydrate disorders, not necessarily accompanied by alterations in the lipid balance. The use of berberine to treat alterations of the lipid balance is described in WO 2007/090289A1 and WO 2007/113748.

However, the oral bioavailability of berberine is low, which limits its efficacy. Its pharmacokinetic characteristics are not yet fully understood. After intravenous administration, berberine presents linear kinetics with an AUC value that increases in proportion to the dose injected. From the blood, it is transferred to the liver, where it is processed and secreted through the hepatobiliary tract. In the liver, berberine is metabolised by a first demethylation stage followed by a second glucuronidation stage. The proportion of berberine detectable in the plasma after oral administration is very low. In the blood, berberine is detectable unchanged or in glucuronidated form. Its main metabolites are berberrubine, talifendine, dimethylene berberine and jatrorrhizine, detectable in the blood in free or glucuronidated form.

### Description of the invention

It has now been found that the oral bioavailability of berberine can be considerably increased by associating it with some phytotherapeutic preparations, , namely extracts of Silybum marianum and especially the flavonolignan fraction obtained by extraction from milk thistle (silymarin). The oral bioavailability of the compound can increase up to 6-8 fold.

As glycoprotein P is abundantly expressed in the intestinal cells, and in view of the evident effects of the first stage associated with protein P-450, the oral bioavailability and absorption of berberine are presumably influenced by co-administration of inhibitors of those protein systems, as demonstrated by the data in our possession. From the kinetic standpoint, however, the most striking finding was obtained by co-administering silymarin. In view of the known hepatoprotective action of said compound and the crucial role played by the liver in carbohydrate disorders, silymarin can be considered an ideal candidate for the treatment of carbohydrate disorders with any etiology, as its use is designed to increase the oral bioavailability of berberine, and extracts containing it and to contribute to their hypoglycaemic effect. However, the validity of the invention does not depend in any way on the verification of that hypothesis.

The disclosure therefore relates to compositions containing:
a) berberine or Berberis aristata extracts containing it
b) Berberine may be present in the compositions according to disclosure as such or in the form of an extract containing it, namely an extract of *Berberis aristata.*

The quantity of berberine per dose unit is between 100 and 1000 mg, while ingredient b) is present in quantities of between 100 and 500 mg per unit dose.

The compositions according to the disclosure can be prepared with conventional techniques and excipients. Examples of dosage forms include tablets (film-coated, gastroprotected, time- or pH-dependent controlled-release, multi-layer), capsules, sachets, granulates, chewable gum discs, and orodispersible forms.

The phytotherapeutic ingredients of the compositions according to the disclosure and in particular the extracts of *Silybum marianum* and silymarin, may be present in phytosome (complexed with phospholipids), liposome, co-ground or ter-clathrate form.

The compositions according to the disclosure may also contain antioxidants such as vitamins (C and E), extracted polyphenols (OPC from *Vitis vinifera,* anthocyanosides and proanthocyanidins from bilberry), cysteine, cystine, methionine, glutathione, lipoic acid and ubidecarenone.

The invention relates to the use of berberine or extracts containing it in association with extracts of *Silybum marianum,* or purified fractions or pure ingredients of said extracts, for the preparation of hypocholesterolaemic and hypoglycaemic compositions.

In particular, the use of berberine and silymarin or extracts of *Silybum marianum* to prepare hypocholesterolaemic and hypoglycaemic compositions is preferred.

The following examples illustrate the invention in greater detail.

### EXAMPLES

### EXAMPLE 1 - Film-coated tablets

| Ingredient | Quantity |
|---|---|
| *Beriberis aristata 97%* | 515 mg |
| *Silybum marianum* dried extract | 210 mg |
| Microcel | 200 mg |
| Dicafos | 200 mg |
| Vegetable magnesium stearate | 40 mg |
| PVP CL | 40 mg |
| Silicon dioxide | 20 mg |
| Shellac | 50 mg |

### EXAMPLE 2 - "0" format capsules

| Ingredient | Quantity |
|---|---|
| *Beriberis aristata 97%* | 515 mg |
| *Silybum marianum* dried extract | 210 mg |
| Talc | 10 mg |
| Microcel 101 | 50 mg |

### EXAMPLE 3 - Sachets

| Ingredient | Quantity |
|---|---|
| *Beriberis aristata 97%* | 1030 mg |
| *Silybum marianum* dried extract | 420 mg |
| Fructose | 1000 mg |
| Methocel E5 | 20 mg |
| Aerosol | 50 mg |
| Acesulfame K sweetener | 10 mg |
| E110 | 2 mg |
| Citrus fruit flavouring | 150 mg |

### EXAMPLE 4 - Sachets

| Ingredient | Quantity |
|---|---|
| *Beriberis aristata 97%* | 515 mg |
| *Silybum marianum* dried extract | 210 mg |
| Saccharose | 2265 mg |
| Citric acid | 50 mg |
| Silicon dioxide | 20 mg |
| Flavouring | 150 mg |
| Acesulfame K | 15 mg |

### EXAMPLE 5 - Programmed-release two-layer tablets

| Ingredient | Quantity |
|---|---|
| NORMAL-RELEASE LAYER | |
| *Beriberis aristata 97%* | 515 mg |
| *Silybum marianum* dried extract | 210 mg |
| Dicafos | 304 mg |
| Aerosil | 3 mg |
| Vegetable magnesium stearate | 6 mg |
| Colouring | 1 mg |
| SLOW-RELEASE LAYER | |
| *Beriberis aristata 97%* | 515 mg |
| *Silybum marianum* dried extract | 210 mg |
| Metholose | 80 mg |
| Aerosil | 2 mg |
| Vegetable magnesium stearate | 3 mg |
| Microcel | 80 mg |
| Dicafos | 164 mg |

### EXAMPLE 6 - Orodispersible formulation

| Ingredient | Quantity |
|---|---|
| *Beriberis aristata 97%* | 515 mg |
| *Silybum marianum* dried extract | 210 mg |
| Sorbitol | 160 mg |
| Orange flavouring | 20 mg |
| Mandarin flavouring | 5 mg |
| Acesulfame K | 2 mg |
| Aerosil | 5 mg |
| Fructose | 1566 mg |

### EXAMPLE 7 - Gum disc

| Ingredient | Quantity |
|---|---|
| *Beriberis aristata 97%* | 100 mg |
| *Silybum marianum* dried extract | 50 mg |
| Gum base | 800 mg |
| Aspartame | 2 mg |
| Menthol | 2 mg |
| Acesulfame | 1 mg |
| Levilite | 20 mg |
| Talc F.U. | 20 mg |
| Vegetable magnesium stearate | 18 mg |
| Shellac | 12 mg |
| Xylitol | 250 mg |
| Gum arabic | 6 mg |
| Titanium dioxide | 6 mg |
| Carnauba wax | 0,2 mg |

## Claims

1. The combination of berberine and silymarin or of *Beriberis aristata* extract containing berberine and of *Silybum marianum* extract containing silymarin for use in the treatment of hyperglycemia.

## Patentansprüche

1. Die Kombination von Berberin und Silymarin oder von Berberin enthaltendem *Berberis* aristata-Extrakt und Silymarin enthaltendem *Silybum* marianum-Extrakt zur Verwendung bei der Behandlung von Hyperglykämie.

## Revendications

1. Combinaison de berbérine et de silymarine, ou d'extrait de *Berberis aristata* contenant de la berbérine et d'extrait de *Silybum marianum* contenant de la silymarine pour une utilisation dans le traitement de l'hyperglycémie.
